# EUROPÄISCHE PATENTSCHRIFT

(11) **EP 1 039 941 B1**
(45) Veröffentlichungstag und Bekanntmachung des Hinweises auf die Patenterteilung: **09.07.2003**
(21) Anmeldenummer: 98966517.9
(22) Anmeldetag: 09.12.1998
(51) Int. Cl.: A61M 5/172, A61M 5/168

(54) **VORRICHTUNG ZUR VERABREICHUNG EINER INFUSION UND/ODER PERFUSION AN EINEN PATIENTEN**
DEVICE FOR GIVING A TRANSFUSION AND/OR PERFUSION TO A PATIENT
DISPOSITIF PERMETTANT D'ADMINISTRER D'UN MEDICAMENT A UN PATIENT PAR PERFUSION

(30) Priorität: 19.12.1997 DE 19756872
(43) Veröffentlichungstag der Anmeldung: 04.10.2000
(73) Patentinhaber: SIEMENS AKTIENGESELLSCHAFT, 80333 München (DE)
(72) Erfinder: ABRAHAM-FUCHS, Klaus, D-91058 Erlangen (DE); BIRKHÖLZER, Thomas, D-91085 Weisendorf (DE); SCHMIDT, Volker, D-91054 Erlangen (DE)
(86) Internationale Anmeldenummer: DE9803620
(87) Internationale Veröffentlichungsnummer: WO99032176

(56) Entgegenhaltungen:
- EP-A- 0 702 966
- WO-A-86/02625
- WO-A-96/32975
- DE-A- 2 326 265
- US-A- 4 392 849
- US-A- 4 526 568
- US-A- 4 922 975

## Beschreibung

Die Erfindung betrifft eine Vorrichtung zur Verabreichung einer Infusion und/oder Perfusion an einen Patienten.

Eine Vorrichtung gemäß dem Oberbegriff von Anspruch 1 ist aus der US-A-4 526 568 bekannt.

In der Intensivmedizin bestehen wesentliche Aufgaben darin, Störungen des Flüssigkeits-, Elektrolyt- und des Energiehaushaltes eines Patienten auszugleichen und den Patienten über eine gewisse Zeit parenteral zu ernähren. Dies wird üblicherweise durch eine Mischung verschiedener Infusionslösungen erreicht, die parenteral über einen zentralvenösen Zugang dem Patienten gegeben werden. Die Zusammensetzung der Infusionslösungen richtet sich nach dem Bedürfnis des Patienten und den zugrunde liegenden Störungen der genannten Haushalte, die ausgeglichen werden müssen. Diese Störungen lassen sich grob unterteilen in eine Überwässerung oder einen Wassermangel, in Störungen der Blutsalzzusammensetzung und in Störungen des Energiestoffwechsels, wobei diese Störungen sich mitunter gegenseitig beinflussen und komplexe Zusammenhänge aufweisen. Die Planung einer Infusionsbehandlung erfordert daher die Berechnung eines individuellen Infusionsplanes.

Der Wasserbedarf wird dabei über messbare Verluste und Abschätzungen betreffend nicht messbare Verluste anhand von Faustregeln bestimmt. Störungen des Wasserhaushaltes werden durch Messungen des Körpergewichts, durch Messung des zentralen Venendrucks, durch Beurteilung des Kreislaufs sowie von Ödemen oder Hautfalten und schließlich durch Bestimmung der Verluste (Urin, Atmung, Schweiß, Stuhlgang) abgeschätzt. Die Kontrolle der Wasserbilanzierung erfolgt über die gleichen Parameter. Anhand dieses grobgeschätzten Wasserbedarfs wird dann die zuzuführende Infusionsmenge pro Zeiteinheit bestimmt. Der Elektrolytbedarf wird über geschätzte Verluste sowie über Elektrolytbestimmungen in Körperflüssigkeiten (Serum, Urin, Flüssigkeiten aus Drainagen) ebenfalls anhand von Faustregeln bestimmt. Störungen der Blutsalzzusammensetzung werden über Laboruntersuchungen des Blutes beurteilt, wobei hier insbesondere die Natrium-, Kalium- und Chloridspiegel im Blut relevant sind, wie aber auch andere Elektrolyte. Schließlich wird auch der Kalorienbedarf ebenfalls zumeist über Faustregeln abgeschätzt, in welche das Körpergewicht des Patienten, die Temperatur und die Krankheit des Patienten eingehen. Daneben ist auch eine Bestimmung des Kalorienbedarfs durch indirekte Kalorimetrie möglich. Die Aufteilung des Kalorienbedarfs auf die Nährstoffe Eiweiß, Kohlenhydrate und Fett, die die zuzuführenden Kalorienträger bilden, erfolgt ebenfalls über Faustregeln. Die Kontrolle der parenteralen Ernährung, also letztlich das Infusionsergebnis, erfolgt über die Messung des Blutzuckers und/oder der Blutfettwerte. Aus den genannnten groben Schätzungen wird für jeden Patienten ein individueller Infusionsplan errechnet, der dann durch Zusammenmischen von Infusionslösungen mit Salzen umgesetzt wird. Diese Lösungen werden dann mit einer ebenfalls von Hand vorgegebenen Infusionsgeschwindigkeit zugeführt. Die Therapie, also das Infusionsergebnis, wird anschließend durch Laborkontrollen überprüft und gegebenenfalls modifiziert.

Die obige Beschreibung zeigt, daß eine Infusionstherapie, wie sie momentan durchgeführt wird, viele Unsicherheitsfaktoren, Nährungen und Annahmen enthält, die eine an die tatsächlichen Erfordernisse angepaßte Infusionstherapie nicht ermöglichen. Insbesondere ist eine kontinuierliche Kontrolle des Therapieergebnisses, also die Auswirkung der Infusion bzw. Perfusion auf den auszugleichenden Haushalt bzw. Parameter nur in groben Zeitabständen möglich, so daß die gesamte Infusions- bzw. Perfusionstherapie äußerst unflexibel ist.

In der DE-AS 28 49 367 ist eine Vorrichtung zur Regelung der Glucosekonzentration im Blutstrom einer Person beschrieben, bei der eine Infusionsvorrichtung veranlaßt wird, dem Blutstrom Insulin in einer Menge zuzuführen, die auf Grundlage einer mathematischen Gleichung ermittelt wird.

In der DE 39 02 497 A1 ist außerdem eine Perfusionsvorrichtung in Form einer Herzunterstützungseinrichtung beschrieben, bei der der Perfusionsvorgang auf Grundlage eines fest vorgegebenen Modells gesteuert wird.

Die Erfindung liegt damit das Problem zugrunde, eine Vorrichtung anzugeben, welche eine verbesserte Infusions- und/oder Perfusionstherapie zuläßt und ein weitgehend auf die tatsächlichen Gegebenheiten abgestimmtes Arbeiten zuläßt.

In der EP 0 702 966 A2 ist ein Dosierungssystem für ein im Zusammenhang mit medizinischen Bildgebungsverfahren erforderliches Kontrastmittel beschrieben, bei dem die Konzentration des Kontrastmittels und die Injektionsparameter patientenspezifisch eingestellt werden, um den Verbrauch von Kontrastmittel zu verringern und die Qualität der Bilder zu erhöhen.

Die US 4 392 849 betrifft eine Steuerung für eine Infusionspumpe mit einer geschlossenen Regelschleife. In der WO 86/02625 ist ein System zum Mischen von Lösungen zur künstlichen Ernährung beschrieben, das mittels eines Computers gesteuert ist.

Zur Lösung dieses Problems ist erfindungsgemäß eine Vorrichtung zur Verabreichung einer Infusion und/oder Perfusion an einen Patienten vorgesehen, umfassend:
- ein oder mehrere Sensormittel zum Messen von Ist-Werten eines oder mehrerer patientenspezifischer Parameter,
- ein mit dem oder den Sensormittel(n) kommunizierendes Steuermittel,
- eine mit dem Steuermittel kommunizierende und die zu verabreichende Infusions- und/oder Perfusionslösung enthaltende Infusions- und/oder Perfusionseinrichtung,
wobei das Steuermittel die mittels der Infusions- und/oder Perfusionseinrichtung zuzuführende Infusions- und/oder Perfusionsmenge in Abhängigkeit der erfaßten Ist-Werte steuert, und
- wobei das Steuermittel (5) ein Expertensystem umfaßt, mittels welchem die eingehenden Ist-Werte verarbeitet werden, und basierend auf welchem die Steuerung erfolgt.

Die erfindungsgemäße Vorrichtung stellt mit besonderem Vorteil ein Regelungssystem dar, welches die Zufuhr an Infusions- und/oder Perfusionslösung abgestimmt auf die tatsächlich vorliegenden Bedingungen, die mittels der Ist-Werte feststellbar sind, ermöglicht. Es erfolgt eine dauernde Kommunikation zwischen dem oder den Sensormitteln mit dem Steuermittel, wobei die Ist-Werte des oder der patientenspezifischen Parametern im wesentlichen kontinuierlich oder quasi kontinuierlich erfaßt werden. Abhängig von den Ist-Werten steuert dann das Steuermittel die Gabe der Infusions- und/oder Perfusionslösungen. Infolge des im wesentlichen kontinuierlichen oder quasi kontinuierlichen Erfassen der Ist-Werte erhält man auf diese Weise sofort eine Rückmeldung über den Erfolg der zugeführten Lösungen, d.h., es wird sehr rasch erkannt, ob die gewünschte Wirkung im Hinblick auf den auszugleichenden Haushalt erreicht wird.

Wie bereits beschrieben handelt es sich bei den auszugleichenden Haushalten bzw. den zu kontrollierenden Störungen jedoch um teilweise sehr komplexe Zusammenhänge, sowohl im Hinblick auf das Auftreten der Störung wie auch deren Auswirkung auf den Gesamtkörper. Im Hinblick auf diese komplexen Zusammenhänge, die aber medizinisch hinreichend erfaßt sind, ist erfindungsgemäß vorgesehen, das daß Steuermittel ein Expertensystem unfaßt, mittels welchem die eingehenden Ist-Werte verarbeitet werden, und basierend auf welchem die Steuerung erfolgt. Bei diesen Expertensystem handelt es sich um ein intelligentes Regelungs- und Steuerungssystem, wobei die Regelungs-und Steuermechanismen basierend auf physiologischen und pathophysiologischen Modellen des Stoffwechsels, also auf den medizinischen Erkenntnissen über die komplexen Zusammenhänge erstellt wurde. Auf diese Weise ist es also mit besonderem Vorteil möglich, die beschriebenen komplexen Zusammenhänge im Rahmen der Steuerung zu berücksichtigen und eine verbesserte Therapie zu ermöglichen. Ein weiterer beachtlicher Vorteil des Einsatzes dieses Expertensystems, welches selbtverständlich in Form einer entsprechenden Regelungs- und Steuerungssoftware steuermittelseitig vorgesehen ist, liegt ferner darin, daß in dieses Expertensystem sämtliche bekannten Zusammenhänge und Informationen aufnehmbar sind, die zur Festlegung der Therapie und damit zur entsprechenden Steuerung erforderlich sind, und die bisher vom Arzt nur aufgrund eigenen Wissens in die Erstellung des Therapieplans einzubringen waren, wobei hierbei selbstverständlich ein beachtliches Fehlerpotential gegeben war, welches mit dem beliebig ausbau- und strukturierbaren Expertensystem beseitigt ist. Die eingangs genannten Probleme treten nicht mehr auf, da die erfindungsgemäße Regelung basierend auf den Ist-Werten, die in dem als Expertensystem ausgebildeten Steuermittel verarbeitet werden, eine wesentlich genauere und schnell nachvollziehbare Infusions- bzw. Perfusionstherapie zulassen.

Wie bereits beschrieben ist es insbesondere im Rahmen der Intensivmedizin erforderlich verschiedene Haushalte zu kontrollieren, so daß unterschiedliche Infusions- oder Perfusionslösungen zuzuführen sind. Um dem Rechnung zu tragen kann in aus der DE 28 55 713 C2 an sich bekannter Weise vorgesehen sein, daß die Infusions- und/oder Perfusionseinrichtung mehrere zu verabreichende Infusions- und/oder Perfusionslösungen enthält, wobei das Steuermittel die Abgabe der einzelnen erforderlichen Infusions- und/oder Perfusionsmengen steuert. Die Vorrichtung ermöglicht also mit besonderem Vorteil, unterschiedlichste Lösungen zu verwenden und zu verabreichen, so daß es für eine Komplettversorgung verwendet werden kann. Dabei kann die Infusions- und/oder Perfusionseinrichtung eine Mischeinrichtung aufweisen, in welcher die über das Steuermittel gesteuert abgegebenen Infusions- und/oder Perfusionslösungen gemischt werden, d.h., daß Zusammenmischen erfolgt gesteuert, die zusammengemischte Lösung wird anschließend über eine gemeinsame Leitung zugeführt, so daß nicht mehrere Katheter gelegt werden müssen. Sämtliche Schritte erfolgen vorteilhaft stets unter Kontrolle des Steuermittels.

Mithin erfordern vom Arzt feststellbare physiologische Parameter oder Zusammenhänge, eine oder mehrere Infsuionsund/oder Perfusionslösungen defizitär, ausgleichend oder überschüssig zuzuführen. Um die Vorrichtung auch für solche Fälle einsetzbar zu gestalten, kann gemäß einer vorteilhaften Weiterbildung des Erfindungsgedankens vorgesehen sein, das daß Steuermittel ein oder mehrere Wählmittel aufweist, über welche (s) die abzugebende Menge einer oder mehrerer Infusions- und/oder Perfusionslösungen benutzerseitig zur Einstellung einer defizitären, ausgeglichenen oder überschüssigen Zufuhr einstellbar ist. Unter "defizitärer, ausgleichender oder überschüssiger Zufuhr" ist sowohl eine entsprechende Zufuhr an Flüssigkeit im Hinblick auf den Flüssigkeits- oder Elektrolythaushalt zu verstehen, wie auch im Falle einer Beeinflussung des Kalorienhaushalts eine hypokalorische, normokalorische und hyperkalorische Zufuhr möglich ist. Dabei hat es sich als zweckmäßig erwiesen, wenn das Steuermittel selbst zur automatischen Einstellung einer defizitären, ausgeglichenen oder überschüssigen Zufuhr ausgebildet ist, um dem Arzt auch diese Entscheidung abnehmen zu können. Die steuermittelseitige Entscheidung diesbezüglich erfolgt in Abhängikeit des Expertensystems und der gelieferten Ist-Werte bzw. der entsprechenden Parameterdaten.

Für den Fall, daß ein Haushalt oder ein patientenspezifischer Parameter mittels unterschiedlicher Infusions- und/oder Perfusionslösungen ausgeglichen werden kann, wie dies beispielsweise für den Kalorienhaushalt gilt, welche durch Zugabe von Fett-, Eiweiß- und/oder Kohlenhydratlösungen beeinflußt werden kann, die jeweils richtige Lösung(en) bzw. richtige Zusammensetzung zu verwenden, kann ferner vorgesehen sein, das daß Steuermittel ein oder mehrere, gegebenenfalls weitere Wählmittel zum Auswählen der Art der zuzuführenden Infusionsund/oder Perfusionslösungen aufweist. Mit Hilfe dieses Wählmittels kann also der Arzt die Zusammensetzung der zuzuführenden Lösung einstellen. Dies kann beispielsweise so sein, daß er bestimmte Zusammensetzungsverhältnisse "Fett/Eiweiß/Kohlenhydrate" wählen kann, welche im Rahmen der Abgabesteuerung entsprechend berücksichtigt werden. Auch hier kann das Steuermittel zum automatischen Auswählen der Art der zuzuführenden Infusions- und/oder Perfusionslösungen ausgebildet sein.

Im Hinblick auf einen reibungslosen und kontinuierlichen Einsatz der Vorrichtung hat es sich ferner als vorteilhaft erwiesen, wenn ein oder mehrere Sensormittel mittels einer in der Infusions- und/oder Perfusionseinrichtung vorgesehenen Eichlösung spülbar und kalibrierbar sind, so daß die Sensormittel stets korrekte Ist-Werte messen und liefern können. Die Spülung und Kalibrierung, die mittels dem Steuermittel steuerbar sein kann, kann mehrmals in vorgegebenen Zeitabständen erfolgen und erfordert lediglich den Einsatz einer Eichlösung im Mikroliter-Bereich, so daß die gegebenenfalls in den Patienten nach dem Spülen eingebrachte Eichlösung die Therapie in keinem Fall beeinflussen kann. Schließlich hat es sich als zweckmäßig erwiesen, wenn eine in Abhängigkeit wenigstens eines gemessenen Ist-Wertes mittels des Steuermittels betätigbare Alarmeinrichtung vorgesehen ist, welche im Falle einer festgestellten Komplikation, in der Regel dann, wenn ein Ist-Wert von einem im Steuermittel abgelegten SollWert erheblich abweicht, eine ensprechende Alarmierung des Personals ermöglicht.

Die Vorrichtung kann zur infusions- und/oder perfusionsgestützen Beeinflussung des Glycose- und/oder Kaliumhaushalts eines Patienten ausgebildet sein. Alternativ oder zusätzlich kann die Vorrichtung zur infusions- und/oder perfusionsgestützten Beeinflussung des Kalorienhaushalts eines Patienten ausgebildet sein, wie auch -alternativ oder zusätzlich- eine Ausbildung der Vorrichtung zur infusions- und/oder perfusionsgestützten Beeinflussung des Flüssigkeits- und/oder Elektrolythaushalts eines Patienten vorgesehen sein kann. Im Hinblick auf ein möglichst breites Einsatzspektrum der erfindugsgemäßen Vorrichtung, was insbesondere in der Intensivmedizin gefordert wird, sollte die Vorrichtung zur Beeinflussung jedes der genannten Haushalte ausgebildet sein.

Gemäß einer Variante der Erfindung werden zusätzlich zu den auszugleichenden oder zu beeinflussenden Parametern (Haushalten) weitere physikalische und/oder chemische Parameter mittels der Sensormittel gemessen werden und von dem Steuermittel verarbeitst, und zwar zumindest ein Teil der folgenden Parameter: Patialdruck O₂ der Ein- und Ausatemluft, Partialdruck CO₂ der Ausatemluft, Raumtemperatur, Luftdruck, Temperatur des Patienten, Kalium-, Natrium- und Chloridspiegel im Blut, pH-Wert des Bluts, Partialdruck O₂ und CO₂ im Blut, Glucosespiegel im Blut, Triglyceridspiegel im Blut, zentraler Venendruck, arterieller Blutdruck, Urinproduktion pro Zeiteinheit, Kreatininspiegel, Körpergewicht.

Die Infusions- und/oder Perfusionseinrichtung kann sich dadurch auszeichnen, daß sie zur Aufnahme mehrerer Infusionsund/oder Perfusionslösungen ausgebildet ist, die über separate Leitungen einer zentralen Ausgabeleitung zuführbar sind, und daß ein oder mehrere den Lösungsfluß beeinflussende Stell- und/oder Pumpelemente vorgesehen sind, die über ein mit der Einrichtung über einen Kommunikationsanschluß verbindbares oder verbundenes Steuermittel steuerbar sind. In die erfindungsgemäße Vorrichtung können also beliebig viele Lösungen, insbesondere Standardlösungen eingebracht, beispielsweise eingehängt werden, die dann an entsprechende separate Leitungen anschließbar sind. Mittels der Stellund/oder Pumpelemente, die jeder Leitung bzw. jeder Infusions- und/oder Perfusionslösung zugeordnet sein können, werden dann die abgegebenen Mengen separat und individuell gesteuert. Des weiteren kann eine Mischeinrichtung zum Mischen der verschiedenen, über die gemeinsame Abgabeleitung zuzuführenden Infusions- und/oder Perfusionslösungen vorgesehen sein, wobei diese Mischeinrichtung als gemeinsamer Tropfenfänger ausgebildet sein kann, in den die einzelnen zuzuführenden Infusion- und/oder Perfusionslösungen tropfenweise gebbar sind.

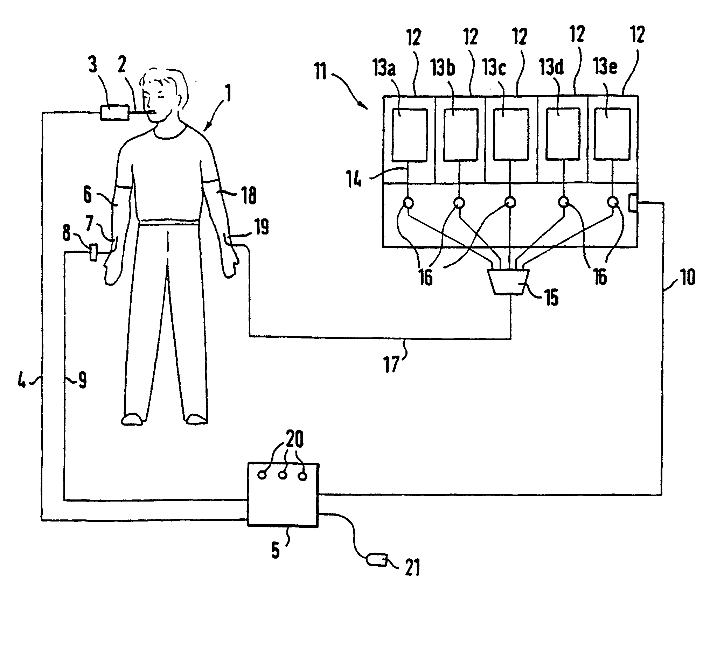

Weitere Vorteile,Neuerungen und Einzelheiten der Erfindung ergeben sich aus dem im folgenden beschriebenen Ausführungsbeispiel sowie anhand der Zeichnung.

Die Figur zeigt in Form einer Prinzipskizze eine erfindungsgemäße Vorrichtung. Gezeigt ist ein Patient 1, welcher durch Gabe einer Infusion oder Perfusion zu therapieren ist. An einem Tubus 2 ist ein Sensormittel 3 vorgesehen, welches der Atemgassensorik dient. Mittels dem Sensormittel 3 können die Partialdrucke des Sauerstoffs und des Kohlendyoxids in der Ausatemluft gemessen werden, wie auch der Partialdruck des Sauerstoffs in der Einatemluft. Über eine Kommunikationsleitung 4 werden die im wesentlichen kontinuierlich oder quasikontinuierlich gemessenen Ist-Werte an ein Steuermittel 5 gegeben. Am Arm 6 des Patienten 1 ist ein arterieller oder venöser Dauerkatheter 7 angeordnet, dem Sensormittel 8 für die Blutsensorik nachgeschaltet sind, die über eine Kommunikationsleitung 9 mit dem Steuermittel 5 verbunden sind. Mittels der Sensormittel 8 können beispielsweise die Kalium-, Natrium- und Chloridspiegel im Blut, der Blut-pH der Sauerstoffpartialdruck und der Kohlensäurepartialdruck im Blut gemessen werden, wie gegebenenfalls auch die Blutglucose- und Triglyceridspiegel. Auch der zentrale Venendruck kann hierüber messbar sein, wie gegebenenfalls auch der arterieller Blutdruck. Auch hier werden kontinuierlich oder quasi kontinuierlich Ist-Werte ermittelt, die dem Steuermittel 5 gegeben werden.

Die Figur zeigt lediglich ein Ausführungsbeispiel. Eine Beschränkung hinsichtlich der einsetzbaren Sensormittel und der mit diesen messbaren Parametern ist hierdurch nicht gegeben. Vielmehr können beliebige Sensormittel zur Messung beliebiger Parameter eingesetzt werden, solang diese für die Steuerung erforderlich sind. Die Sensormittel sollten ein möglichst geringes Probenvolumen erfordern, um die Belastung des Patienten möglichst gering zu halten, beispielsweise hinsichtlich des erforderlichen Blutverbrauchs für die Sensorik. Auch sollte die Baugröße möglichst klein sein, da auf Intensivstationen zumeist Platzmangel herrscht. Insbesondere im Hinblick auf den Blutkontakt sollte der Preis gering sein, da es sich um ein Wegwerfprodukt handeln muß. Schließlich sollte auch die Lebensdauer der Sensormittel nach Möglichkeit der Behandlungsdauer entsprechen, damit nicht ein dauernder Austausch erforderlich ist. Die Sensormittel können ferner mittels einer Eichlösung spülbar und kalibrierbar sein, wobei dies über das Steuermittel gesteuert erfolgen kann.

Über eine Kommunikationsleitung 10 steht das Steuermittel 5 mit einer Infusions- und/oder Perfusionseinrichtung 11 in Kommunikationsverbindung. Die Einrichtung 11 weist mehrere Aufnahmen 12 zur Aufnahme unterschiedlicher Infusionsund/oder Perfusionslösungen 13 a,b,c,d,e auf, wobei im gezeigten Beispiel lediglich fünf Aufnahmen vorgesehen sind, jedoch beliebig viele vorgesehen sein können. Bei den Lösungen 13 a-e handelt es sich um Standardlösungen. Jede Lösung 13 a-e ist über eine Leitung 14 mit einer Mischeinrichtung 15 in Form eines Tropfenfängers verbunden. In jede Leitung 14 ist ein Stell- und/oder Pumpelement 16 geschalten, wobei jedes Stell- und/oder Pumpelement 16 in seinem Betrieb über das Steuermittel 5 gesteuert wird. D.h., hierüber kann die abzugebende Menge der jeweiligen Lösung 13 a-e gesteuert werden. Da dem Steuermittel 5 die ist-Werte aufgrund der Sensorik bekannt sind, kann auf diese Weise umgehend auf entsprechende Änderungen reagiert werden und durch Ansteuern des jeweiligen Stell- und/oder Pumpelements die zugegebene Menge der jeweiligen Lösung variiert werden.

Jede abgegebene Lösung wird tropfenweise über die entsprechende Leitung 14 der Mischeinrichtung 15 zugegeben, wo die einzelnen Lösungen miteinander vermischt werden. Über die Leitung 17 wird die in ihrer Zusammensetzung auf die tatsächlichen Ist-Gegebenheiten abgestimmte Lösung dem Patienten am Arm 18 über einen entsprechenden Katheter 19 zugegeben.

Wie bereits beschrieben erfolgt die gesamte Steuerung der Einrichtung 11 über das Steuermittel 5. Zu diesem Zweck ist das Steuermittel 5 das als Steuerrechner ausgebildet ist, mit einem Expertensystem in Form einer Regelungs- und Steuerungssoftware versehen. Das Expertensystem wurde auf Basis physiologischer und pathophysiologischer Stoffwechselmodelle konfiguriert und dient zur Verarbeitung der gelieferten Ist-Werte, um hieraus den entsprechenden Steuerungsplan zu generieren.

Wie bereits beschrieben sind die einsetzbaren Sensormittel nicht begrenzt. Beispielsweise kann die gesamte Vorrichtung als Glucose- und Kalium-System ausgebildet sein. In diesen Fall wird mittels eines entsprechenden Sensormittels im Blut der Kalium- und der Glucosespiegel bestimmt und durch die Regelung der Einrichtung 11, die in diesen Fall zumindest eine Glucose- und eine Kaliumlösung enthält, in einem konstanten Bereich gehalten, wobei zusätzlich ein Insulinperfusor zum Einsatz kommen kann.

Alternativ (oder zusätzlich) kann die Vorrichtung auch kalorienbedarfsgesteuert sein. In diesen Fall wird über das Verfahren die indirekten Kalorimetrie der aktuelle Kalorienverbrauch des Patienten bestimmt (Kalorien/Minute). Die Einrichtung 11 enthält in diesen Fall (gegebenenfalls zusätzlich) Elektrolyt-, Kohlenhydrat-, Aminosäurelösungen und Fettemulsionen, wie auch fertige Mischungen hieraus eingesetzt werden können. Zusätzlich kann auch hier ein Insulinperfusor vorgesehen sein. Durch entsprechende Steuerung/Regelung über das Steuermittel 5 wird dem Patienten eine adäquate Kalorienmenge zugeführt. Hierbei kann zwischen einer hypokalorischen, einer normokalorischen und einer hyperkalorischen Ernährung gewählt werden, wozu geeigneterweise entsprechende Wählmittel- und Steuermittel 5 (in der Figur durch die Schalter 20 dargestellt) vorgesehen sein können. Über die jeweiligen festgestellten Kalorienverbräuche kann auch festgestellt werden, was der Patient verbrennt, d.h., ob er bevorzugt Fette verbrennt, oder aber Kohlenhydrate, so daß die Zusammensetzung entsprechend eingestellt werden kann. Die Auswahl kann aber auch automatisch vom Steuermittel gesteuert erfolgen. Auch können mittels der Wählmittel 20 nur bestimmte zu verabreichende Lösungen oder gegebenenfalls bestimmte Mischungsverhältnisse eingestellt werden. Zur Sicherheit kann hier zusätzlich noch der Blutglucosespiegel, der Kaliumspiegel wie auch der Natriumspiegel und der zentrale Venendruck gemessen werden. Eine Volumenüberlastung des Patienten kann durch Erfassung des zentralen Venendrucks und der Urinproduktion ermittelt werden.

Schließlich kann die Vorrichtung auch zur Regelung des Wasser- und Elektrolythaushalts ausgebildet sein. Über das Körpergewicht, die Temperatur, die Verluste (Drainagen, Urin, Stuhlgang)und den zentralen Venendruck wird von dem Steuermittel 5 die Menge Wasser berechnet, die der Patient benötigt. Auch hier kann der Arzt beispielsweise mittels der Wählmittel 20 wählen, ob ein Defizit, ein genauer Ausgleich oder ein Überschuß an Volumen erzielt werden soll, einschließlich des gewünschten Grades. Im Blut werden über geeignete Sensormittel die Elektrolyte, Natrium, Kalium, ect. bestimmt. Die Menge und die Zusammensetzung der Infusionslösung bezüglich ihres Salzgehalts werden dann mittels des Steuermittels berechnet. Zusätzlich kann noch die Nierenfunktion über den Kreatininspiegel ermittelt werden.

Schließlich ist eine Alarmeinrichtung 21 vorgesehen, mittels welcher im Falle einer Komplikation ein Alarm gegeben werden kann.

## Patentansprüche

1. Vorrichtung zur Verabreichung einer Infusion und/oder Perfusion an einen Patienten, umfassend:
- ein oder mehrere Sensormittel (3, 8) zum Messen von Ist-Werten eines oder mehrerer patientenspezifischen Parameter,
- ein mit dem oder den Sensormittel(n) (3, 8) kommunizierendes Steuermittel (5),
- eine mit dem Steuermittel (5) kommunizierende und die zu verabreichende Infusions- und/oder Perfusionslösung enthaltende Infusions- und/oder Perfusionseinrichtung (11),
- wobei das Steuermittel (5) die mittels der Infusionsund/oder Perfusionseinrichtung (11) zuzuführende Infusionsund/oder Perfusionsmenge in Abhängigkeit der erfaßten Ist-Werte steuert, **dadurch gekennzeichnet, daß**
- das Steuermittel (5) ein Expertensystem umfaßt, mittels welchem die eingehenden Ist-Werte verarbeitet werden, und basierend auf welchem die Steuerung erfolgt.

2. Vorrichtung nach Anspruch 1, bei der die Infusionsund/oder Perfusionseinrichtung (11) mehrere zu verabreichende Infusions- und/oder Perfusionslösungen (13a, b, c, d, e) enthält, wobei das Steuermittel die Abgabe der einzelnen erforderlichen Infusions- und/oder Perfusionsmengen steuert.

3. Vorrichtung nach Anspruch 2, bei der die Infusionsund/oder Perfusionseinrichtung (11) eine Mischeinrichtung (15) aufweist, in welcher die über das Steuermittel gesteuert abgegebenen Infusions- und/oder Perfusionslösungen (13a, b, c, d, e) gemischt werden.

4. Vorrichtung nach einem der vorangehenden Ansprüche, bei der das Steuermittel (5) ein oder mehrere Wählmittel (20) aufweist, über welche(s) die abzugebende Menge einer oder mehrerer Infusions- und/oder Perfusionslösungen (13a, b, c, d, e) benutzerseitig zur Einstellung einer defizitären, ausgeglichenen oder überschüssigen Zufuhr einstellbar ist.

5. Vorrichtung nach einem der Ansprüche 1 bis 3, bei der das Steuermittel (5) zur automatischen Einstellung einer defizitären, ausgeglichenen oder überschüssigen Zufuhr ausgebildet ist.

6. Vorrichtung nach einem der vorangehenden Ansprüche, bei der das Steuermittel (5) ein oder mehrere, gegebenenfalls weitere Wählmittel (20) zum Auswählen der Art der zuzuführenden Infusions- und/oder Perfusionslösungen (13a, b, c, d, e) aufweist.

7. Vorrichtung nach einem der Ansprüche 1 bis 5, bei der das Steuermittel (5) zum automatischen Auswählen der Art der zuzuführenden Infusions- und/oder Perfusionslösungen (13a, b, c, d, e) ausgebildet ist.

8. Vorrichtung nach einem der vorangehenden Ansprüche, bei der ein oder mehrere Sensormittel (3, 8) mittels einer in der Infusions- und/oder Perfusionseinrichtung (11) vorgesehenen Eichlösung spülbar und kalibrierbar sind.

9. Vorrichtung nach Anspruch 8, bei der das Spülen und Kalibrieren mittels dem Steuermittel (5) steuerbar ist.

10. Vorrichtung nach einem der vorangehenden Ansprüche, bei der eine in Abhängigkeit wenigstens eines gemessenen Ist-Wertes mittels des Steuermittels (5) betätigbare Alarmeinrichtung (21) vorgesehen ist.

11. Vorrichtung nach einem der vorangehenden Ansprüchen, welche zur infusions- und/oder perfusionsgestützten Beeinflussung des Glucose- und/oder Kaliumhaushalts eines Patienten ausgebildet ist.

12. Vorrichtung nach einem der vorangehenden Ansprüche, welche zur infusions- und/oder perfusionsgestützten Beeinflussung des Kalorienhaushalts eines Patienten ausgebildet ist.

13. Vorrichtung nach einem der vorangehenden Ansprüche, welche zur infusions- und/oder perfusionsgestützten Beeinflussung des Flüssigkeits- und/oder Elektrolythaushalts eines Patienten ausgebildet ist.

14. Vorrichtung nach einem der Ansprüche 1 bis 13, deren Infusions- und/oder Perfusionseinrichtung zur Aufnahme mehrerer Infusions- und/oder Perfusionslösungen (13a, b, c, d, e) ausgebildet ist, die über separate Leitungen (14) einer zentralen Ausgabeleitung (17) zuführbar sind, wobei ein oder mehrere den Lösungsfluß beeinflussende Stell- und/oder Pumpelemente (16) vorgesehen sind, die über ein mit der Einrichtung (11) über einen Kommunikationsanschluß verbindbares oder verbundenes Steuermittel (5) steuerbar sind.

15. Vorrichtung nach Anspruch 14, bei deren Infusionsund/oder Perfusionseinrichtung jeder Leitung (14) bzw. jeder Infusions- und/oder Perfusionslösung (13a, b, c, d, e) ein eigenes Stell- und/oder Pumpelement (16) zugeordnet ist.

16. Vorrichtung nach Anspruch 14 oder 15, deren Infusionsund/oder Perfusionseinrichtung eine Mischeinrichtung (17) zum Mischen der verschiedenen, über die gemeinsame Abgabeleitung (17) zuzuführenden Infusions- und/oder Perfusionslösungen (13a, b, c, d, e) aufweist.

17. Vorrichtung nach Anspruch 16, deren Mischeinrichtung (17) als gemeinsamer Tropfenfänger ausgebildet ist, in den die einzelnen zuzuführenden Infusions- und/oder Perfusionslösungen (13a, b, c, d, e) tropfenweise gebbar sind.

18. Vorrichtung nach einem der Ansprüche 1 bis 17, bei welchem mittels der Sensormittel (3, 8) zumindest ein Teil der folgenden Parameter gemessen und mittels des Steuermittels (11) verarbeitet wird:
Partialdruck O₂ in der Ein- und Ausatemluft, Partialdruck CO₂ der Ausatemluft, Raumtemperatur, Luftdruck, Temperatur des Patienten, Kalium-, Natrium- und Chloridspiegel im Blut, pH-Wert des Bluts, Partialdruck O₂ und CO₂ im Blut, Glucosespiegel im Blut, Triglyceridspiegel im Blut, zentraler Venendruck, arterieller Blutdruck, Urinproduktion pro Zeiteinheit, Kreatininspiegel, Körpergewicht.

## Claims

1. Device for administering an infusion and/or perfusion to a patient, comprising:
- one or more sensor means (3, 8) for measuring real values of one or more patient-specific parameters,
- a control means (5) that communicates with the one or more sensor means (3, 8),
- an infusion device and/or perfusion device (11) which communicates with the control means (5) and which contains the infusion solution and/or perfusion solution to be administered,
- whereby the control means (5) controls the infusion amount and/or perfusion amount to be supplied by means of the infusion device and/or perfusion device (11) dependent on the acquired real values, **characterised in that**
- the control means (5) comprises an expert system by means of which the incoming real values are processed and on the basis of which the control is carried out.

2. Device according to Claim 1, whereby the infusion device and/or perfusion device (11) contains a plurality of infusion solutions and/or perfusion solutions (13a, b, c, d, e) to be administered, whereby the control means controls the output of the individual required infusion amounts and/or perfusion amounts.

3. Device according to Claim 2, whereby the infusion device and/or perfusion device (11) comprises a mixing device (15), in which the infusion solutions and/or perfusion solutions (13a, b, c, d, e) output via the control means in a controlled manner are mixed.

4. Device according to one of the preceding claims, whereby the control means (5) comprises one or more selection means (20), via which the amount of one or more infusion solutions or perfusion solutions (13a, b, c, d, e) to be output can be adjusted at the user side to adjust a deficient supply, balanced supply or excess supply.

5. Device according to one of Claims 1 to 3, whereby the control means (5) is designed to automatically adjust a deficient supply, balanced supply or excess supply.

6. Device according to one of the preceding claims, whereby the control means (5) comprises one or more selection means (20), potentially further selection means (20), for selecting the type of the infusion solutions and/or perfusion solutions (13a, b, c, d, e) to be supplied.

7. Device according to one of Claims 1 to 5, whereby the control means (5) is designed to automatically select the type of the infusion solutions and/or perfusion solutions (13a, b, c, d, e) to be supplied.

8. Device according to one of the preceding claims, whereby one or more sensor means (3, 8) can be rinsed and calibrated by means of a calibrating solution provided in the infusion device and/or perfusion device (11).

9. Device according to Claim 8, whereby the rinsing and calibrating can be controlled by means of the control means (5).

10. Device according to one of the preceding claims, whereby an alarm device (21) is provided which can be operated by means of the control means (5) depending on at least one measured real value.

11. Device according to one of the preceding claims which is designed to influence the glucose metabolism and/or potassium metabolism of a patient in an infusion-supported manner and/or perfusion-supported manner.

12. Device according to one of the preceding claims which is designed to influence the caloric metabolism of a patient in an infusion-supported manner and/or perfusion-supported manner.

13. Device according to one of the preceding claims which is designed to influence the fluid metabolism and/or electrolyte metabolism of a patient in an infusion-supported manner and/or perfusion-supported manner.

14. Device according to one of Claims 1 to 13, whose infusion device and/or perfusion device is designed to accept a plurality of infusion solutions and/or perfusion solutions (13a, b, c, d, e), which can be supplied via separate lines (14) of a central output line (17), whereby one or more control elements and/or pumping elements (16) that influence the solution flow are provided, which can be controlled via a control means (5) that can be connected or is connected to the device (11) via a communication connection.

15. Device according to Claim 14, whose infusion device and/or perfusion device has a separate control element and/or pumping element (16) allocated to each line (14) or, respectively, to each infusion solution and/or perfusion solution (13a, b, c, d, e).

16. Device according to Claim 14 or 15, whose infusion device and/or perfusion device comprises a mixing device (17) for mixing the different infusion solutions and/or perfusion solutions (13a, b, c, d, e) to be supplied via the common output line (17).

17. Device according to Claim 16, whose mixing device (17) is designed as a common drop catcher, into which the individual infusion solutions and/or perfusion solutions (13a, b, c, d, e) to be supplied can be given drop-by-drop.

18. Device according to one of Claims 1 to 17, whereby at least one part of the following parameters is measured by means of the one or more sensor means (3, 8) and is processed by means of the control means (11):
Partial pressure O₂ in the inspired air and expired air, partial pressure CO₂ in the expired air, room temperature, air pressure, temperature of the patient, potassium level, sodium level, chloride level in the blood, pH-value of the blood, partial pressure O₂ and CO₂ in the blood, glucose level in the blood, triglyceride level in the blood, central venous pressure, arterial blood pressure, urine production per time unit, creatinine level, body weight.

## Revendications

1. Dispositif d'administration à un patient par goutte à goutte ou par perfusion comprenant :
- un ou plusieurs moyens (3, 8) formant capteurs destinés à mesurer des valeurs réelles d'un paramètre ou de plusieurs paramètres spécifiques au patient,
- un moyen (5) de commande communiquant avec le ou les moyens (3, 8) formant capteurs,
- un dispositif (11) de goutte à goutte ou de perfusion communiquant avec le moyen (5) de commande et contenant la solution de goutte à goutte ou de perfusion à administrer,
- le moyen (5) de commande commandant la quantité de goutte à goutte et de perfusion à envoyer au moyen du dispositif (11) de goutte à goutte ou de perfusion en fonction des valeurs réelles détectées, **caractérisé en ce que**
- le moyen (5) de commande comprend un système expert au moyen duquel les valeurs réelles entrées sont traitées et sur la base duquel s'effectue la commande.

2. Dispositif suivant la revendication 1, dans lequel le dispositif (11) de goutte à goutte ou de perfusion contient plusieurs solutions (13a, b, c ,d ,e) de goutte à goutte ou de perfusion à administrer, le moyen de commande commandant la sortie des quantités de goutte à goutte ou de perfusion nécessaires individuellement.

3. Dispositif suivant la revendication 2, dans lequel le dispositif (11) de goutte à goutte ou de perfusion comprend un dispositif (15) de mélange dans lequel les solutions (13a, b, c, d, e) de goutte à goutte ou de perfusion fournies de manière commandée par le moyen de commande sont mélangées.

4. Dispositif suivant l'une des revendications précédentes, dans lequel le moyen (5) de commande a un ou plusieurs moyens (20) de sélection par lequel ou par lesquels les quantités fournies d'une ou de plusieurs solutions (13a, b, c, d, e) de goutte à goutte ou de perfusion peuvent être réglées par l'utilisateur pour établir un apport déficitaire, de compensation ou en excès.

5. Dispositif suivant l'une des revendications 1 à 3, dans lequel le moyen (5) de commande est constitué pour établir automatiquement un apport déficitaire, de compensation ou en excès.

6. Dispositif suivant l'une des revendications précédentes, dans lequel le moyen (5) de commande a un ou plusieurs moyens (20) éventuellement supplémentaires de sélection de la nature des solutions (13a, b, c, d, e) de goutte à goutte ou de perfusion à apporter.

7. Dispositif suivant l'une des revendications 1 à 5, dans lequel le moyen (5) de commande est constitué pour sélectionner automatiquement la nature des solutions (13a, b, c, d, e) de goutte à goutte ou de perfusion à apporter.

8. Dispositif suivant l'une des revendications précédentes, dans lequel un ou plusieurs moyens (3, 8) formant capteurs peuvent être lavés et/ou étalonnés au moyen d'une solution d'étalonnage prévue dans le dispositif (11) de goutte à goutte ou de perfusion.

9. Dispositif suivant la revendication 8, dans lequel le lavage et l'étalonnage peuvent être commandés à l'aide du moyen (5) de commande.

10. Dispositif suivant l'une des revendications précédentes, dans lequel il est prévu un dispositif (21) d'alerte qui peut être actionné à l'aide du moyen (5) de commande en fonction d'au moins une valeur réelle mesurée.

11. Dispositif suivant l'une des revendications précédentes, qui est constitué pour influencer par goutte à goutte ou par perfusion le bilan du glucose et/ou du potassium d'un patient.

12. Dispositif suivant l'une des revendications précédentes, qui est constitué pour influencer par goutte à goutte ou par perfusion le bilan calorique d'un patient.

13. Dispositif suivant l'une des revendications précédentes, qui est constitué pour influencer par goutte à goutte ou par perfusion le bilan des liquides et/ou des électrolytes d'un patient.

14. Dispositif suivant l'une des revendications 1 à 13, dont le dispositif de goutte à goutte ou de perfusion est constitué pour la réception de plusieurs solutions (13a, b, c, d, e) de goutte à goutte ou de perfusion qui peuvent être apportées par des conduits (14) distincts à un conduit (17) central de sortie, dans lequel il est prévu un ou plusieurs éléments (16) de réglage et/ou de pompage qui influencent le débit de solution et qui peuvent communiquer avec le dispositif (11) par un raccord de mise en communication ou qui peuvent être commandés par le moyen (5) de commande qui est relié.

15. Dispositif suivant la revendication 14, dans le dispositif de goutte à goutte ou de perfusion duquel il est associé à chaque conduit (14) ou à chaque solution (13a, b, c, d, e) de goutte à goutte ou de perfusion un élément (16) propre de réglage et/ou de pompage.

16. Dispositif suivant la revendication 14 ou 15, dont le dispositif de goutte à goutte ou de perfusion comporte un dispositif (17) de mélange des diverses solutions (13a, b, c, d, e) de goutte à goutte ou de perfusion à apporter par le conduit commun de sortie.

17. Dispositif suivant la revendication 16, dont le dispositif (17) de mélange est constitué en capteur commun de gouttes, dans lequel les solutions (13a, b, c, d, e) à apporter individuellement peuvent être fournies goutte à goutte.

18. Dispositif suivant l'une des revendications 1 à 17, dans lequel on mesure à l'aide du moyen (3, 8) formant capteur au moins une partie des paramètres suivants et on les traite à l'aide du moyen (11) de commande :
pression partielle d'O₂ dans l'air inspiré et expiré, pression partielle de CO₂ de l'air expiré, température ambiante, pression de l'air, température du patient, niveau du potassium, du sodium et du chlore dans le sang, pH du sang, pression partielle d'O₂ et de CO₂ dans le sang, niveau du glucose dans le sang, niveau des triglycérides dans le sang, pression veineuse centrale, pression sanguine artérielle, production d'urine par unité de temps, niveau de créatinine, poids corporel.
